Europäisches Patentamt

(19) European Patent Office

Office européen des brevets

(11) EP 0 680 784 B1

(12) **FASCICULE DE BREVET EUROPEEN**

(45) Date de publication et mention
de la délivrance du brevet:
**07.07.1999 Bulletin 1999/27**

(51) Int. Cl.$^6$: **B01J 27/053**, B01J 31/02, C07C 2/62

(21) Numéro de dépôt: **95400905.6**

(22) Date de dépôt: **24.04.1995**

(54) **Catalyseur d'alkylation d'isoparaffine C4-C5 par au moins une oléfine C2-C6**

Katalysator zur Alkylierung eines C4-C5 isoparaffins mit mindestens einem C2-C6 Olefin

Catalyst for alkylating a C4-C5 isoparaffin with at least a C2-C6 olefin

(84) Etats contractants désignés:
**DE GB IT NL**

(30) Priorité: **04.05.1994 FR 9405492**

(43) Date de publication de la demande:
**08.11.1995 Bulletin 1995/45**

(73) Titulaire:
**INSTITUT FRANCAIS DU PETROLE**
**92502 Rueil-Malmaison (FR)**

(72) Inventeurs:
- **Benazzi, Eric**
  **F-78360 Montesson (FR)**
- **Joly, Jean-François**
  **F-69006 Lyon (FR)**
- **Marcilly, Christian**
  **F-78800 Houilles (FR)**

(56) Documents cités:
EP-A- 0 433 954          EP-A- 0 539 277
EP-A- 0 542 612          US-A- 5 233 119

**Description**

La présente invention concerne un catalyseur comprenant un support poreux organique ou minéral, de préférence de la silice, et une phase acide comportant le composé $B(OSO_2CF_3)_3$ et au moins un acide choisi dans le groupe formé par l'acide sulfurique ($H_2SO_4$) et l'acide trifluorométhanesulfonique ($CF_3SO_3H$), la silice ayant été imprégnée par ladite phase acide. L'invention concerne aussi la préparation et l'utilisation dudit catalyseur en alkylation catalytique d'isoparaffine (isobutane et/ou isopentane) en présence d'au moins une oléfine comportant de 2 à 6 atomes de carbone par molécule.

Pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation d'isoparaffine (isobutane et/ou isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation, qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent généralement soit l'acide sulfurique soit l'acide fluorhydrique comme catalyseur. Dans ces procédés le catalyseur acide constitue une phase liquide qui est mise en contact avec le mélange isobutane-oléfine(s) liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes, tels que les tamis moléculaires, les résines macroréticulaires éventuellement associées avec $BF_3$, les acides de Lewis et/ou de Brönsted déposés sur divers supports inorganiques, les alumines chlorées, les graphites intercalés par des acides de Lewis et/ou de Brönsted et les anions déposés sur supports oxydes tels que $ZrO_2/SO_4$. Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés), et donc une régénération fréquente. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

La demande de brevet européen EP-A-0539277 décrit un catalyseur contenant de la silice et une phase acide solide comprenant de l'acide sulfurique, la silice selon ladite demande de brevet est telle que son volume poreux est compris entre 0,005 et 1,5 $cm^3/g$, et sa surface spécifique est comprise entre 0,01 et 1500 $m^2/g$. Ladite phase acide comprend éventuellement un additif choisi dans le groupe formé par $H_3PO_4$, $B(OH)_3$, $BF_4H$, $FSO_3H$, $CF_3CO_2H$, $SbF_5$, $CF_3SO_3H$ et $SO_3$.

La demande de brevet français de numéro d'enregistrement national 93/04.735 (& EP-A 623 388, publiée 9.11.94) décrit un catalyseur comprenant un support poreux organique ou minéral et le mélange constitué par l'acide sulfurique, l'acide trifluorométhane sulfonique et éventuellement l'eau.

La présente invention concerne un catalyseur comprenant un support poreux organique ou minéral, de préférence de la silice, et une phase acide comportant le composé $B(OSO_2CF_3)_3$ et au moins un acide du groupe formé par l'acide sulfurique ($H_2SO_4$) et l'acide trifluorométhane sulfonique ($CF_3SO_3H$), ledit support ayant été imprégné par ladite phase acide, ledit catalyseur étant tel qu'il est constitué essentiellement de particules de diamètre moyen compris entre 0,1 et 150 $\mu m$ (1 $\mu m$ = $10^{-6}m$) de préférence entre 5 et 110 $\mu m$ et de manière encore plus préférée entre 5 et 80 $\mu m$, ledit catalyseur étant caractérisé en ce que ladite phase acide comprend :

- entre 0,1 et 70 % poids, et de préférence entre 0,2 et 65% poids, de $B(OSO_2CF_3)_3$;
- entre 0 et 90 % poids, et de préférence entre 0 % et 80 % poids, de $H_2SO_4$;
- entre 0 et 90 % poids, et de préférence entre 0 % et 85 %, de $CF_3SO_3H$.

L'invention concerne aussi la préparation et l'utilisation dudit catalyseur en alkylation catalytique d'au moins une isoparaffine choisi dans le groupe formé par l'isobutane et l'isopentane (c'est-à-dire l'isobutane et/ou l'isopentane : l'isobutane, ou l'isopentane, ou l'isobutane et l'isopentane) en présence d'au moins une oléfine comportant de 2 à 6 atomes de carbone par molécule, de préférence de 3 à 6 atomes de carbone par molécule.

Le catalyseur selon la présente invention conduit de façon surprenante à des performances catalytiques améliorées par rapport à celles décrites dans la demande de brevet européen EP-A-0539277 et dans la demande de brevet

2

français de numéro d'enregistrement national 93/04.735 (& EP-A 623 388, publiée 9.11.94).

Le titre de l'acide sulfurique est avantageusement compris entre 90 et 100% poids, de préférence entre 97 et 100% poids et de manière encore plus préférée entre 98 et 100% poids.

Le titre de l'acide trifluorométhane sulfonique est avantageusement compris entre 95 et 100% poids et de préférence entre 98 et 100% poids.

La teneur pondérale du catalyseur selon l'invention en phase acide est généralement supérieure à 40 %, de préférence supérieure à 70 %

Le composé $B(OSO_2CF_3)_3$ compris dans la phase acide du catalyseur selon l'invention est préparé selon les méthodes connues de l'homme du métier. Par exemple et à titre non limitatif, on peut citer les méthodes préférées selon l'invention, la première méthode préférée selon l'invention consistant à faire réagir un trihalogénure de bore $BX_3$ (X étant un halogène, de préférence X est Cl ou Br), avec 3 équivalents molaires d'acide trifluorométhane sulfonique ($CF_3SO_3H$) selon la réaction :

$$BX_3 + 3\ CF_3SO_3H \rightarrow B(OSO_2CF_3)_3 + 3HX$$

la seconde méthode préférée slon l'invention consisitant à faire réagir une mole de trihalogénure de bore $BX_3$ (X étant un halogène, de préférence X est Cl ou Br), avec 3 équivalents molaires de trifluorométhane sulfonate d'argent $CF_3SO_3Ag$ :

$$BX_3 + 3\ CF_3SO_3Ag \rightarrow B(OSO_2CF_3)_3 + 3AgX$$

Les deux réactions précédentes, liées aux première et seconde méthodes préférées selon l'invention, sont décrites en particulier par Engelbrecht et al. dans Z. Anorg. Chem., **1977,** *433*, 19 et par G. A Olah et al. dans J. Org. Chem. **1984,** *49*, 4591-4594.

Lorsque la silice est utilisée comme support, elle peut contenir des impuretés comme par exemple les oxydes, les alcalins, les alcalinoterreux, les composés d'aluminium ou toute autre impureté connue de l'homme du métier, la quantité totale de ces impuretés n'excédant généralement pas 2 % en poids par rapport à la silice Le support poreux organique ou minéral, de préférence la silice, est généralement tel que, avant son imprégnation par la phase acide, sa surface spécifique est comprise entre 0,1 et 1 500 $m^2$/g, et son volume poreux total est compris entre 0,1 et 6 $cm^3$/g, de préférence entre 0,35 et 4,5 $cm^3$/g et de manière encore plus préférée entre 1,5 et 4 $cm^3$/g. De plus, il est généralement constitué essentiellement de particules de diamètre moyen compris entre 0,1 et 150 µm, de préférence entre 5 et 110 µm, et de manière encore plus préférée entre 5 et 80 µm.

La phase acide occupe généralement entre 80 et 100 % du volume poreux total du support, et de préférence entre 90 et 100 % dudit volume poreux.

Le procédé de préparation du catalyseur selon l'invention comprend deux étapes. Dans une première étape le support poreux organique ou minéral est calciné à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination est généralement effectuée en présence d'air sec ou de mélange air sec/azote, de débit compris entre 0,001 et 10 l/h/g, de préférence entre 0,1 et 5 l/h/g. La seconde étape consiste en l'imprégnation dudit support calciné, par la phase acide. Pour réaliser cette étape on peut utiliser toutes les techniques bien connues de l'homme du métier. A ce procédé de préparation peut s'ajouter une étape de préparation de la phase acide, préalable à l'étape d'imprégnation.

Le catalyseur selon la présente invention est mis en oeuvre dans un procédé qui permet de réaliser dans les meilleures conditions la réaction d'alkylation de l'isobutane par les oléfines. En particulier, ladite réaction étant caractérisée par une forte exothermicité (environ 83,6 kJ/mol de butène transformé si l'oléfine est le butène et si l'isoparaffine est l'isobutane), la mise en oeuvre du catalyseur selon la présente invention permet d'obtenir une bonne homogénéité de température et de concentration en réactifs.

Dans le procédé d'alkylation de l'isobutane utilisant le catalyseur selon la présente invention, les conditions opératoires, et plus particulièrement la température et la pression, sont généralement choisies de façon à ce que le mélange constitué par l'isopraffine, l'(les) oléfine(s) et les produits de la réaction soit liquide. De plus, il est important que le catalyseur soit immergé dans ledit liquide de façon à assurer un bon contact liquide-solide.

Le catalyseur selon l'invention est avantageusement mis en oeuvre dans la zone réactionnelle d'alkylation de l'isobutane et/ou de l'isopentane avec au moins une oléfine comprenant de 2 à 6 atomes de carbone par molécule, de préférence de 3 à 6 atomes de carbone par molécule, en phase liquide et en mélange avec l'isoparaffine et/ou un mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur pouvant être utilisé sous forme de suspension selon les deux mises en oeuvre décrites ci-après.

Une première mise en oeuvre préférée du catalyseur selon la présente invention est la zone réactionnelle à mélange presque parfait, c'est-à-dire à mélange parfait ou s'en rapprochant (cuve agitée ou Grignard), utilisant au moins un moyen d'agitation par exemple au moins une hélice, de façon à obtenir une agitation suffisante du catalyseur en suspension dans la phase liquide hydrocarbonée, laquelle comprend généralement l'isoparaffine (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre préférée du catalyseur selon la présente invention en suspension dans une phase hydrocarbonée est le lit mobile à co-courant c'est-à-dire le lit circulant. Dans cette mise en oeuvre le catalyseur en suspension dans la phase liquide hydrocarbonée, comprenant généralement l'isoparaffine (isobutane et/ou isopentane), au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction, est généralement recyclée après séparation de l'alkylat, soit par introduction directe dans la zone réactionnelle, soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 $h^{-1}$ et de préférence comprise entre 0,002 et 2 $h^{-1}$. Ledit mélange peut aussi être réalisé à l'intérieur de la zone réactionnelle. Dans tous les cas le mélange ainsi constitué est dans la zone réactionnelle dans des conditions de pression et de température telles que le mélange d'hydrocarbures reste liquide sur le catalyseur.

La température de réaction est généralement inférieure à +10 °C, de préférence à 0 °C et de manière souvent plus préférée inférieure à -3 °C. La pression de la zone réactionnelle est généralement suffisante pour maintenir les hydrocarbures à l'état liquide dans ladite zone.

Afin de limiter les réactions secondaires, on peut utiliser un excès d'isoparaffine par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Les produits de la réaction peuvent être contrôlés régulièrement par mesure de l'indice de brome, par exemple selon le projet de Norme Française Pr. M. 07.071 de mars 1969.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

## Exemple 1

*Préparation du catalyseur 1 : conforme à l'invention*

On active 16 g de silice macroporeuse de surface spécifique égale à 395 $m^2$/g et de volume poreux total égal à 2,4 $cm^3$/g et de diamètre moyen des particules égal à 45 $\mu$m, par calcination sous air pendant 4 heures à 500 °C. La silice ainsi activée est conservée sous argon. On procède alors à une imprégnation à sec, et à l'abri de l'humidité, de 14 g de ladite silice déshydratée par 44 g du mélange constitué par :

- 37,4 g d'une solution contenant 99 % poids d'$H_2SO_4$ et 1 % poids d'eau,
- 6,6 g d'une solution contenant 6,46 g d'acide $CF_3SO_3H$ et 0,14 g d'eau,
- 6 g du composé $B(OSO_2CF_3)_3$.

La composition pondérale de la phase acide est la suivante :

| - H2SO4 | 74,05 % |
| - $CF_3SO_3H$ | 4,08 % |
| - $B(OSO_2CF_3)_3$ | 12,00 % |
| - H2O | 1,01 % |

Le solide ainsi obtenu, appelé catalyseur 1, possède une teneur pondérale en phase acide égale à 75,9 % poids ; il est conservé sous argon à -18 °C.

*Alkylation de l'isobutane par le butène-1 avec le catalyseur 1*

Le catalyseur 1 est utilisé pour alkyler l'isobutane par le butène-1 de façon à produire des paraffines ramifiées à hauts indices d'octane. On introduit 36 g du catalyseur 1, préparé ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20 °C.

100 $cm^3$ d'isobutane sont alors ajoutés dans le réacteur contenant le catalyseur sous agitation, ledit réacteur étant immergé dans un bain froid à -6 °C. On laisse sous agitation le système catalyseur + isobutane pendant une durée de 30 minutes afin d'homogénéiser la température.

On ajoute en continu un mélange d'isobutane et de butène-1, contenant 20 % poids de butène-1, pendant une durée totale de 8 heures, la température du réacteur étant maintenu à -5 °C pendant toute la durée de l'injection. Le débit volumique du butène-1 est égal à 25 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1. La conversion de l'oléfine est de 100 %.

## Exemple 2

*Préparation du catalyseur 2 non conforme à l'invention*

On prépare 14 g de silice deshydratée d'une façon identique à la préparation du catalyseur conforme à l'invention. On procède alors à une imprégnation à sec, et à l'abri de l'humidité, de 14 g de ladite silice par 44 g du mélange constitué par.

- 17,4 g d'une solution contenant 99 % poids d'$H_2SO_4$ et 1 % poids d'eau,
- 6,6 g d'une solution contenant 6,46 g d'acide $CF_3SO_3H$ et 0,14 g d'eau.

La composition pondérale de la phase acide est la suivante :

| - $H_2SO_4$ | 84,15 % |
| - $CF_3SO_3H$ | 14,68 % |
| - $H_2O$ | 1,17 % |

Le solide ainsi obtenu, appelé catalyseur 2, possède une teneur pondérale en phase acide égale à 75,9 % poids ; il est conservé sous argon à -18 °C.

*Alkylation de l'isobutane par le butène-1 avec le catalyseur 2*

Le catalyseur 2 est utilisé pour alkyler l'isobutane par le butène-1 de façon à produire des paraffines ramifiées à hauts indices d'octane. On introduit 36 g du catalyseur 2, préparé ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20 °C.

100 cm$^3$ d'isobutane sont alors ajoutés dans le réacteur contenant le catalyseur sous agitation, ledit réacteur étant immergé dans un bain froid à -6 °C. On laisse sous agitation le système catalyseur 2 + isobutane pendant une durée de 30 minutes afin d'homogénéiser la température.

On ajoute en continu un mélange d'isobutane et de butène-1, contenant 20 % poids de butène-1, pendant une durée totale de 8 heures, la température du réacteur est maintenu à -5 °C pendant toute la durée de l'injection. Le débit volumique du butène-1 est égal à 10 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1. La conversion de l'oléfine est de 100 %.

Tableau 1

|  | CATALYSEUR 1 Exemple 1 | CATALYSEUR 2 Exemple 2 |
|---|---|---|
| C$_5$-C$_7$ | 2,1 | 2,5 |
| C$_8$ total | 94,1 | 93 |
| C$_9^+$ | 3,8 | 4,5 |

Le tableau 1 met en évidence l'effet de la présence du composé B(OSO$_2$CF$_3$)$_3$ dans le catalyseur. Le catalyseur 1, conforme à l'invention, conduit, pour un débit de butène-1 deux fois et demi plus grand que celui utilisé pour tester le catalyseur 2 non conforme à l'invention, a une sélectivité en composés C$_8$ supérieure à celle obtenue en présence du catalyseur 2. Le catalyseur 1, conforme à l'invention, est donc plus actif et plus sélectif que le catalyseur 2.

## Exemple 3

*Préparation du catalyseur 3 conforme à l'invention*

On active 15 g de silice de volume poreux total égal à 2,2 cm$^3$/g, de surface spécifique égale à 420 m$^2$/g et de diamètre moyen des particules égal à 60 μm, par séchage à 150°C pendant 12 heures. La silice ainsi activée est conservée sous azote. On procède alors à une imprégnation à sec de 10 g de ladite silice par 40,5 g du mélange constitué par ;

- 33,2 g d'une solution d'H$_2$SO4 à 100 % poids en H$_2$SO$_4$,
- 7,3 g du composé B(OSO$_2$CF$_3$)$_3$.

La composition pondérale de la phase acide est la suivante :

| - H$_2$SO$_4$ | 82,0 % |
|---|---|
| - B(OSO$_2$CF$_3$)$_3$ | 18,0 % |

Le solide ainsi obtenu est appelé catalyseur 3, possède une teneur pondérale en phase acide égale à 80,2 % poids ; il est conservé à l'abri de l'humidité sous argon à -18°C.

*Alkylation de l'isobutane par le butène-1 avec le catalyseur 3*

On introduit 20 g du catalyseur 3 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm$^3$ d'isobutane sont alors ajoutés dans le réacteur contenant le catalyseur sous agitation (hélice), ledit réacteur étant immergé dans un bain froid à - -5°C. On laisse sous agitation le système catalyseur 3 + isobutane pendant une durée de 30 minutes afin d'homogénéiser la température.

On ajoute régulièrement 6,1 g de butène-1 par heure pendant une durée totale de 6 heures, la température du réacteur est maintenu à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2. La conversion de l'oléfine est de 100 %.

## Exemple 4

*Préparation du catalyseur 4 non conforme à l'invention*

On prépare 10 g de silice deshydratée, identique à celle employée dans l'exemple 3, d'une façon identique à la préparation du catalyseur conforme à l'invention de l'exemple 3 ci-dessus. On procède alors à un imprégnation à sec, et à l'abri de l'humidité, de 10 g de ladite silice par 40,5 g d'acide sulfurique $H_2SO_4$ (100 % poids).

Le solide ainsi obtenu est appelé catalyseur 4, possède une teneur pondérale en phase acide égale à 80,2 % poids ; il est conservé à l'abri de l'humidité sous argon à -18°C.

*Alkylation de l'isobutane par le butène-1 avec le catalyseur 4*

On introduit 20 g du catalyseur 4 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm$^3$ d'isobutane sont alors ajoutés dans le réacteur contenant le catalyseur sous agitation (hélice), ledit réacteur étant immergé dans un bain froid à -5°C. On laisse sous agitation le système catalyseur 4 + isobutane pendant une durée de 30 minutes afin d'homogénéiser la température.

On ajoute régulièrement 6,1 g de butène-1 par heure pendant une durée totale de 6 heures, la température du réacteur est maintenu à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2. La conversion de l'oléfine est de 100 %.

Tableau 2

|  | CATALYSEUR 3 Exemple 3 | CATALYSEUR 4 Exemple 4 |
|---|---|---|
| $C_5$-$C_7$ | 3,3 | 8,7 |
| $C_8$ total | 92,5 | 80,5 |
| $C_9^+$ | 4,2 | 10,8 |

Le tableau 2 met en évidence l'effet de la présence du composé $B(OSO_2CF_3)_3$ dans le catalyseur. Le catalyseur 3, conforme à l'invention, conduit, pour un même débit de butène-1 à une sélectivité en composés $C_8$ supérieure à celle obtenue en présence du catalyseur 4, non conforme à l'invention. Le catalyseur 3, conforme à l'invention, est donc plus actif et plus sélectif que le catalyseur 4.

## Exemple 5

*Préparation du catalyseur 5 conforme à l'invention*

On active 15 g de silice de volume poreux total égal à 2,2 cm$^3$/g, de surface spécifique égale à 420 m$^2$/g et de diamètre moyen des particules égal à 60 μm, par séchage à 150°C pendant 12 heures. La silice ainsi activée est conservée sous azote. On procède alors à une imprégnation à sec de 10 g de ladite silice par 40,5 g du mélange constitué par ;

- 33,1 g d'une solution CF3SO3H à 100 % poids,
- 7,3 g du composé $B(OSO_2CF_3)_3$.

La composition pondérale de la phase acide est la suivante :

| - $CF_3SO_3H$ | 81,9 % |
|---|---|
| - $B(OSO_2CF_3)_3$ | 18,1 % |

Le solide ainsi obtenu est appelé catalyseur 5, possède une teneur pondérale en phase acide égale à 80,2 % poids ; il est conservé à l'abri de l'humidité sous argon à -18°C.

*Alkylation de l'isobutane par le butène-1 avec le catalyseur 5*

On introduit 20 g du catalyseur 3 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 $cm^3$ d'isobutane sont alors ajoutés dans le réacteur contenant le catalyseur sous agitation (hélice), ledit réacteur étant immergé dans un bain froid à -5°C. On laisse sous agitation le système catalyseur 5 + isobutane pendant une durée de 30 minutes afin d'homogénéiser la température.

On ajoute régulièrement 8 g de butène-1 par heure pendant une durée totale de 6 heures, la température du réacteur est maintenu à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 3. La conversion de l'oléfine est de 100 %.

Tableau 3

| | CATALYSEUR 5 Exemple 5 |
|---|---|
| $C_5$-$C_7$ | 4,3 |
| $C_8$ total | 89,1 |
| $C_9^+$ | 6,6 |

**Revendications**

1.  Catalyseur comprenant un support poreux organique ou minéral et une phase acide comportant le composé $B(OSO_2CF_3)_3$ et au moins un acide choisi dans le groupe formé par l'acide sulfurique ($H_2SO_4$) et l'acide trifluorométhanesulfonique ($CF_3SO_3H$), ledit support ayant été imprégné par ladite phase acide, ledit catalyseur étant tel qu'il est constitué essentiellement de particules de diamètre moyen compris entre 0,1 et 150 $\mu$m, ledit catalyseur étant caractérisé en ce que ladite phase acide comprend :

    - entre 0,1 et 70 % poids de composé $B(OSO_2CF_3)_3$;
    - entre 0 et 90 % poids de $H_2SO_4$;
    - entre 0 et 90 % poids de $CF_3SO_3H$.

2.  Catalyseur selon la revendication 1 tel qu'il est constitué essentiellement de particules de diamètre moyen compris entre 5 et 110 $\mu$m.

3.  Catalyseur selon l'une des revendications 1 ou 2 tel que la teneur pondérale dudit catalyseur en phase acide est supérieure à 40%.

4.  Catalyseur selon l'une des revendications 1 à 3 tel que ledit support est la silice.

**5.** Préparation du catalyseur selon l'une des revendications 1 à 4 comprenant au moins deux étapes, la première étape étant telle que le support est calciné à une température supérieure à 50 °C pour une durée comprise entre 10 minutes et 50 heures, et la deuxième étape étant telle que ledit support calciné est imprégné par ladite phase acide.

**6.** Préparation selon la revendication 5 dans laquelle le composé $B(OSO_2CF_3)_3$ est obtenu selon l'une des méthodes a) ou b) suivantes :

a) en faisant réagir un trihalogénure de bore $BX_3$ (X étant un halogène), avec 3 équivalents molaires d'acide trifluorométhane sulfonique ($CF_3SO_3H$) selon la réaction :

$$BX_3 + 3\ CF_3SO_3H \rightarrow B(OSO_2CF_3)_3 + 3HX.$$

b) en faisant réagir une mole de trihalogénure de bore $BX_3$ (X étant un halogène), avec 3 équivalents molaires de trifluorométhane sulfonate d'argent $CF_3SO_3Ag$ :

$$BX_3 + 3\ CF_3SO_3Ag \rightarrow B(OSO_2CF_3)_3 + 3AgX.$$

**7.** Utilisation du catalyseur selon l'une des revendications 1 à 4 ou préparé selon l'une des revendications 5 ou 6 en alkylation catalytique d'au moins une isoparaffine choisi dans le groupe formé par l'isobutane et l'isopentane en présence d'au moins une oléfine comportant de 2 à 6 atomes de carbone par molécule

**8.** Utilisation selon la revendication 6 dans laquelle la température de réaction est inférieure à +10 °C et la pression de la zone réactionnelle est suffisante pour maintenir les hydrocarbures à l'état liquide dans ladite zone.

**9.** Utilisation selon l'une des revendications 7 à 8 dans laquelle le catalyseur est mis en oeuvre dans une zone réactionnelle à mélange presque parfait.

**10.** Utilisation selon l'une des revendications 7 à 8 dans laquelle le catalyseur est mis en oeuvre en lit mobile à co-courant.

**Claims**

**1.** A catalyst comprising a porous organic or mineral support and an acidic phase comprising $B(OSO_2CF_3)_3$ and at least one acid from the group formed by sulphuric acid ($H_2SO_4$) and trifluoromethane sulphonic acid ($CF_3SO_3H$), said support being impregnated with said acidic phase, said catalyst being essentially constituted by particles with an average diameter of between 0.1 and 150 $\mu$m, said catalyst being characterised in that said acidic phase comprises:

- between 0.1 and 70% by weight of $B(OSO_2CF_3)_3$;
- between 0 and 90% by weight of $H_2SO_4$;
- between 0 and 90% by weight of $CF_3SO_3H$.

**2.** A catalyst according to claim 1, essentially constituted by particles with an average diameter of between 5 and 110 $\mu$m.

**3.** A catalyst according to claim 1 or claim 2, in which the concentration of acidic phase in the catalyst is greater than 40% by weight.

**4.** A catalyst according to any one of claims 1 to 3, in which said support is silica.

**5.** The preparation of a catalyst according to any one of claims 1 to 4, comprising at least two steps, in which in the first step the support is calcined at a temperature of more than 50°C for a period of between 10 minutes and 50 hours, and in the second step said calcined support is impregnated with said acidic phase.

**6.** Preparation according to claim 5, in which the $B(OSO_2CF_3)_3$ is obtained using one of methods (a) or (b) as follows:

a) reacting a boron trihalide $BX_3$ (where X is a halogen) with 3 molar equivalents of trifluoromethane sulphonic

acid ($CF_3SO_3H$) in accordance with the reaction:

$$BX_3 + 3\ CF_3SO_3H \rightarrow B(OSO_2CF_3)_3 + 3\ HX$$

b) reacting one mole of boron trihalide $BX_3$ (where X is a halogen) with 3 molar equivalents of silver trifluoromethane sulphonate $CF_3SO_3Ag$:

$$BX_3 + 3\ CF_3SO_3Ag \rightarrow B(OSO_2CF_3)_3 + 3\ AgX$$

7. The use of a catalyst according to any one of claims 1 to 4 or prepared in accordance with claim 5 or claim 6, for the catalytic alkylation of at least one isoparaffin selected from the group formed by isobutane and isopentane in the presence of at least one olefin containing 2 to 6 carbon atoms per molecule.

8. Use according to claim 6, in which the reaction temperature is less than +10°C and the pressure in the reaction zone is sufficient to maintain the hydrocarbons in the liquid state in said zone.

9. Use according to claim 7 or claim 8, in which the catalyst is used in the reaction zone as an almost perfect mixture.

10. Use according to claim 7 or claim 8, wherein the catalyst is used in a mobile bed in co-current mode.

**Patentansprüche**

1. Katalysator umfassend

   einen porösen organischen oder minderalischen Träger und eine saure Phase, die die Verbindung $B(OSO_2CF_3)_3$ und wenigstens eine Säure umfaßt, die aus der Gruppe gewählt ist, welche gebildet ist durch Schwefelsäure ($H_2SO_4$) und Trifluormethansulfonsäure ($CF_3SO_3H$), wobei dieser Träger durch diese saure Phase imprägniert ist, dieser Katalysator derart ist, daß er im wesentlichen aus Partikeln von einem mittleren Durchmesser zwischen 0,1 und 150 $\mu$m gebildet ist und der Katalysator sich dadurch auszeichnet, daß diese saure Phase umfaßt:

   - zwischen 0,1 und 70 Gew.-% der Verbindung $B(OSO_2CF_3)_3$;
   - zwischen 0 und 90 Gew.-% von $H_2SO_4$ und
   - zwischen 0 und 90 Gew.-% von $CF_3SO_3H$.

2. Katalysator nach Anspruch 1, dadurch gekennzeichnet, daß er im wesentlichen aus Partikeln von einem mittleren Durchmesser zwischen 5 und 110 $\mu$m gebildet ist.

3. Katalysator nach einem der Ansprüche 1 oder 2, derart, daß der Gewichtsgehalt dieses Katalysators an saurer Phase über 40 % beträgt.

4. Katalysator nach einem der Ansprüche 1 bis 3, derart, daß dieser Träger Siliciumoxid ist.

5. Herstellung des Katalysators nach einem der Ansprüche 1 bis 4, wenigstens zwei Stufen umfassend, wobei die erste Stufe derart ist, daß der Träger bei einer Temperatur oberhalb 50 °C für eine Dauer kalziniert wird, die zwischen 10 Minuten und 50 Stunden liegt und die zweite Stufe derart ist, daß dieser kalzinierte Träger durch diese saure Phase imprägniert wird.

6. Herstellung nach Anspruch 5, bei der die Verbindung $B(OSO_2CF_3)_3$ erhalten wird nach einem der folgenden Verfahren a) oder b):

   a) indem man ein Bortrihalogenid $BX_3$ (X ist ein Halogen) mit 3 Moläquivalenten Trifluormethansulfonsäure ($CF_3SO_3H$) nach der Reaktion reagieren läßt:

   $$BX_3 + 3\ CF_3SO_3H \rightarrow B(OSO_2CF_3)_3 + 3HX.$$

   b) indem man ein Mol von Bortrihalogenid $BX_3$ (X ist ein Halogen) mit 3 Moläquivalenten von Silbertrifluormethansulfonat $CF_3SO_3Ag$ reagieren läßt:

$$BX_3 + 3\,CF_3SO_3Ag \rightarrow B(OSO_2CF_3)_3 + 3AgX.$$

7. Verwendung des Katalysators nach einem der Ansprüche 1 bis 4 oder hergestellt nach einem der Ansprüche 5 oder 6 bei der katalytischen Alkylierung wenigstens eines Isoparaffins, gewählt aus der Gruppe, die durch Isobutan und Isopentan in Anwesenheit wenigstens eines Olefins gebildet ist, welches 2 bis 6 Kohlenstoffatome pro Molekül umfaßt.

8. Verwendung nach Anspruch 6, bei dem die Reaktionstemperatur unter +10 °C liegt und der Druck der Reaktionszone ausreichend ist, um die Kohlenwasserstoffe im flüssigen Zustand in dieser Zone zu halten.

9. Verwendung nach einem der Ansprüche 7 oder 8, bei der der Katalysator in einer Reaktionszone mit fast vollkommenem Gemisch eingesetzt wird.

10. Verwendung nach einem der Ansprüche 7 bis 8, bei der der Katalysator im beweglichen Gleichstrombett eingesetzt wird.